# EUROPEAN PATENT APPLICATION

(11) **EP 4 091 627 A1**
(43) Date of publication of application: **23.11.2022**
(21) Application number: 21175229.0
(22) Date of filing: 21.05.2021
(51) Int. Cl.: A61K 39/00, A61K 48/00, C07K 14/47, C07K 14/725, C07K 16/30

(54) **TCR CONSTRUCTS SPECIFIC FOR MAGEA4-DERIVED EPITOPES**

(71) Applicant: Max-Delbrück-Centrum für Molekulare Medizin in der Helmholtz-Gemeinschaft, 13125 Berlin (DE)
(72) Inventor: Lorenz, Felix, 13129 Berlin (DE); Clauß, Julian, 13086 Berlin (DE); Edes, Inan, 13127 Berlin (DE); Stahn, Rainer, 13125 Berlin (DE); Sada Japp, Alberto, 10439 Berlin (DE)
(74) Representative: Moré, Solveig Helga

(57) **Abstract**

The present invention relates to the field of immunotherapy, in particular, of cancer, more specifically, to adoptive T cell therapy or T cell receptor (TCR) gene therapy directed to an epitope derived from the cancer-testis anitgen MAGEA4 presented on HLA-A*01, in particular, the immunodominant epitope of SEQ ID NO: 1. The invention provides a nucleic acid encoding a TCR alpha chain construct (TRA) and/or a TCR beta chain construct (TRB) of a TCR construct, wherein the TCR construct is capable of specifically binding to said epitope in the context of HLA-A*01. Proteins encoded by said nucleic acids, corresponding host cells and pharmaceutical compositions, e.g., for use in treating cancer such as melanoma, gastric cancer, head and neck, lung, breast, ovarian, bladder, and esophageal cancer, are also objects of the invention. In another aspect, the invention provides specific peptides comprising said epitopes, and pharmaceutical compositions comprising the same, e.g., for use in vaccination.

## Description

The present invention relates to the field of immunotherapy, in particular, of cancer, more specifically, to adoptive T cell therapy or T cell receptor (TCR) gene therapy directed to an epitope derived from the cancer-testis anitgen MAGEA4 presented on HLA-A*01, in particular, the immunodominant epitope of SEQ ID NO: 1. The invention provides a nucleic acid encoding a TCR alpha chain construct (TRA) and/or a TCR beta chain construct (TRB) of a TCR construct, wherein the TCR construct is capable of specifically binding to said epitope in the context of HLA-A*01. Proteins encoded by said nucleic acids, corresponding host cells and pharmaceutical compositions, e.g., for use in treating cancer such as melanoma, gastric cancer, head and neck, lung, breast, ovarian, bladder, and esophageal cancer, are also objects of the invention. In another aspect, the invention provides specific peptides comprising said epitopes, and pharmaceutical compositions comprising the same, e.g., for use in vaccination.

A TCR is a heterodimeric cell surface protein of the immunoglobulin super-family which is associated with invariant proteins of the CD3 complex involved in mediating signal transduction. TCRs exist in αβ and γδ forms, which are structurally similar, but have quite distinct anatomical locations and probably functions. The alpha and beta chains of native heterodimeric αβTCR are transmembrane proteins, which each comprise two extracellular domains, a membrane-proximal constant domain, and a membrane-distal variable domain. Each of the constant and variable domains includes an intra-chain disulfide bond.

The variable region of each TCR chain comprises variable and joining segments, and in the case of the beta chain also a diversity segment. Each variable region comprises three CDRs (Complementarity Determining Regions), highly polymorphic loops embedded in framework sequences, one being the hypervariable region named CDR3. There are several types of alpha chain variable (Vα) regions and several types of beta chain variable (Vβ) regions, distinguished by their CDR1 and CDR2 sequences, and by a hypervariable CDR3 sequence. Unique TRAV or TRBV numbers are given to Vα or Vβs by IMGT nomenclature. TCR specificity for the epitopes recognized is mainly determined by the CDR3 regions (Danska et al., 1990; Garcia et al., 2005.).

The use of adoptive T cell therapy allows equipping the patients' own T cells with desired specificities and generation of sufficient numbers of activated, non-exhausted T cells in a short period of time. A specific TCR may be transduced into all T cells or T-cell subsets such as CD8+ T cells, CD4+ T cells, central memory T cells or T cells with stem cell characteristics, which may ensure better persistence and function upon transfer. TCR-engineered T cells may be infused into patients, e.g., cancer patients that have, e.g., been rendered lymphopenic by chemotherapy or irradiation, inducing homeostatic expansion which greatly enhances engraftment and long term persistance of transferred T cells and is associated with higher cure rates.

T cells are able to recognize epitopes presented by MHC (major histocompatibility complex, in humans, also designated HLA, human leukocyte antigen) molecules at the cell surface of cells, such as tumor cells, through their TCR. The function of MHC molecules is the presentation of short peptides (epitopes) derived from proteins (antigens) degraded in the proteasome. T cells can therefore not only detect proteins at the cell surface, but also peptides derived from intracellular antigens. There is a high variety of different MHC alleles expressed by different individuals. Understanding which epitopes are presented by which MHC molecules, and which of those epitopes can be targeted by T cells are critical parameters for the development of epitope-targeting therapeutics.

Thus, a T cell expressing a specific TCR specific for an epitope in the context of a specific MHC can only be used for treatment of a patient expressing the respective MHC allele.

A major impediment for the reliable identification of T cell epitopes is the low specificity of state-of-the-art epitope prediction algorithms. Current algorithms can predict binding affinity of epitopes to HLAs, but they perform poorly when predicting whether epitopes are endogenously processed and presented by tumor cells. Studies have shown that >95% of predicted epitopes are not found at the cell surface of tumor cells (Abelin et al., 2017; Ritz et al., 2018).

An even more critical problem is that it remains unclear which of the few presented epitopes are finally able to induce a T cell response and can thus be targeted with immunotherapies. Comprehensive datasets on immunogenicity of epitopes are lacking so far.

Cancer-testis antigens (CTAs) are highly promising targets for immunotherapy as they offer a safe expression profile and are shared by many patients. Several CTAs of the MAGE family have been described to be safe targets for T cell therapy as their expression in patients is restricted to tumor cells and male germline cells, the latter being protected from the immune system by their lack of HLA expression and the blood-testis barrier. Hence, CTAs are tumor-specific and their targeting with TCRs circumvents the risk of on-target off-tumor toxicity.

The CTA melanoma-associated antigen 4 (MAGEA4) is expressed at high levels in many different types of solid tumors like melanoma, gastric cancer, head and neck, lung, breast, ovarian, bladder, and esophageal cancer (lura et al., 2017; Ishihara et al., 2020; Kobayashi et al., 2003; Ottaviani et al., 2006) and can be recognized by T cells (Kobayashi et al., 2003; Juretic et al., 2003). Thus, TCR-engineered T cell therapy for the treatment of MAGEA4-positive (MAGEA4⁺) tumors presents a unique opportunity for a highly effective cancer treatment with broad applicability.

E.g., in current clinical studies with MAGEA4/HLA-A*02:01 TCRs (Adaptimmune corporate deck, 2020; Immatics corporate presentation, 2021; Immatics press release, 2021; WO 2021023658 A1; WO 2017174824 A1), MAGEA4 is deemed an ideal target for adoptive T cell therapy of solid tumors. Furthermore, it has been described that some adult individuals do not express MAGEA4 or MAGEA1 in their thymus and, hence, central tolerance mechanisms may not exclude functional T cells targeting these antigens from the repertoire of these individuals (Gotter et al., 2004). Thus, highly functional MAGEA4-specific TCRs may be isolated from the peripheral blood of healthy individuals with appropriate technologies.

The identification of TCRs which are restricted to other HLA alleles, e.g., HLA-A1 such as HLA-A*01:01, which is the second most frequent HLA allele in Europe, expressed by more than 25% of the population, and a highly prevalent allele in many other populations, may be a novel treatment option for MAGEA4-associated cancers in populations that are HLA-A*02:01-negative.

The present invention addresses some of these problems, and provides novel, and preferably, advantageous immunotherapeutic agents useful for immunotherapy of cancer, in particular, in HLA-A1 positive subjects. This problem is solved by the subject matter of the claims.

### Nucleic acids encoding TCR constructs

In one embodiment, the invention provides TCR constructs useful for therapy of MAGEA4-positive tumors, and nucleic acids encoding them. In particular, the invention provides the first TCR constructs capable of targeting an epitope of MAGEA4 in complex with HLA-A*01:01.

The invention thus provides a nucleic acid encoding a TCR alpha chain construct (TRA) and/or a TCR beta chain construct (TRB) of a TCR construct specific for an epitope of MAGEA4 in complex with a human MHC I, wherein the MHC I is HLA-A*01:01.

The invention in particular provides a nucleic acid encoding a TCR alpha chain construct (TRA) and/or a TCR beta chain construct (TRB) of a TCR construct specific for an epitope from MAGEA4 in complex with a human MHC I, wherein the epitope has the sequence of SEQ ID NO: 1, the MHC I is HLA-A*01:01, and the TRA comprises a CDR3 having at least 84% sequence identity to SEQ ID NO: 4 and/or the TRB comprises a CDR3 having at least 88% sequence identity to SEQ ID NO: 7.

The preferred TRA CDR3 of SEQ ID NO: 4 consists of 13 amino acids. Thus, at least 84% sequence identity means that at most two amino acids can be substituted (e.g., by conservative substitution), deleted or inserted, preferably, substituted. Preferably, the TRA comprises a CDR3 having at least 92% sequence identity to SEQ ID NO: 4. Most preferably, said CDR3 has SEQ ID NO: 4.

The preferred TRB CDR3 of SEQ ID NO: 7 consists of 18 amino acids. Thus, at least 88% sequence identity means that at most two amino acids can be substituted (e.g., by conservative substitution), deleted or inserted, preferably, substituted. Preferably, the TRA comprises a CDR3 having at least 94% sequence identity to SEQ ID NO: 7. Most preferably, said CDR3 has SEQ ID NO: 7.

In the context of the present invention, "a" is understood to mean "one or more" unless expressly stated otherwise. Accordingly, for example, if the TCR construct of the invention contains both alpha and beta chain constructs, as preferred throughout the invention, it may be encoded by either one or two nucleic acids. The alpha and beta chain constructs together are capable of specifically binding to an epitope from a MAGEA4 protein in complex with a human MHC I, HLA-A*01:01. As intermediate products, the alpha and beta chain constructs and the nucleic acids encoding them are also subject matter of the invention by themselves. The invention also provides a single chain nucleic acid construct, wherein, e.g., TCR alpha and beta chain constructs are separated by a P2A element.

SEQ ID NO: 1 is an epitope from MAGEA4 which the inventors showed to be an immunodominant epitope presented in the context of HLA-A1, e.g., HLA-A*01:01, i.e., a very common HLA-type. It was also presented by all tested MAGEA4-positive tumor lines, and T cells expressing the TCR constructs of the invention were confirmed to recognize these tumor cells.

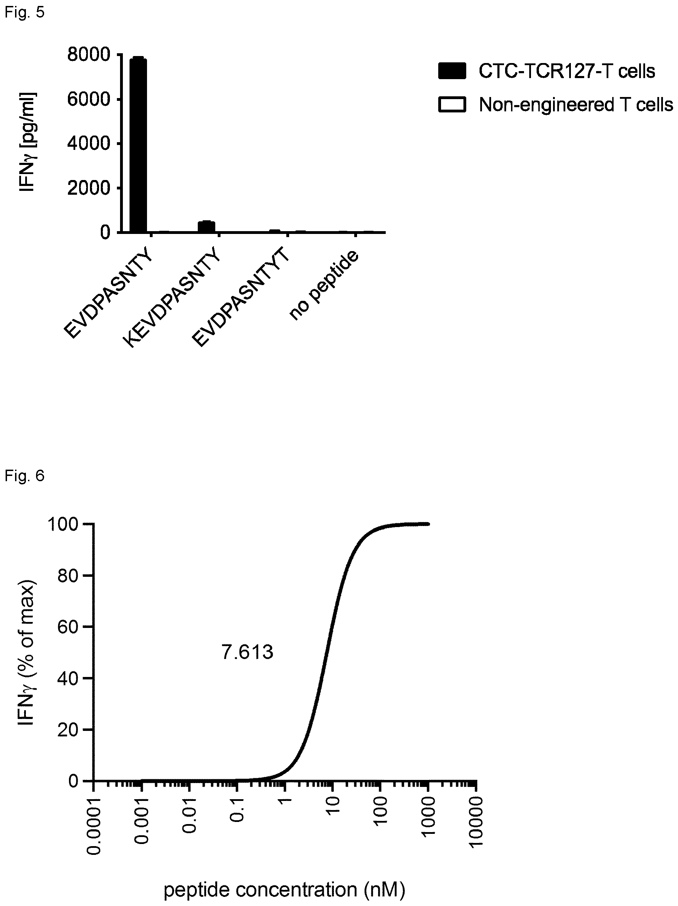

Further, TCR constructs of the invention having the CDR3 sequences disclosed herein, in particular, preferred TCR constructs disclosed herein, have a high functional avidity, as shown herein, compared to other TCRs known in the art. In particular, T cells expressing the TCR constructs of the invention preferably have an EC50 of at most 100 nM, at most 20 nM, preferably, at most 10 nM, e.g., about 7-8 nM. The EC50 value may be determined based on a titration of the peptide of SEQ ID NO: 1 on K562 cells expressing HLA-A*01:01, wherein 5x10⁴ K562 cells are used and 1x10⁵ TCR-transduced T cells are used, and IFNγ release after 18 hour coculture is determined by ELISA, e.g., as carried out for the experiment underlying Fig. 6. The transduction rate, i.e., the fraction of T cells expressing CD8 and CTC-TCR127 after transduction should be at least 50%, preferably, as in the experiments shown herein, at least 65-80%.

In the experiments performed herein, peptide titration was done on K562 cells instead of commonly used T2 cells which carry the HLA-A*02:01 allele. Peptide titration assays on T2 cells may, in general, lead to different results that are not comparable with the EC50 value determined by peptide titration on K562 cells as T2 e.g. express the costimulatory molecule CD80, which leads a higher functional avidity of TCR-T cells through co-receptor binding at the immunological synapse. K562 cells do not express CD80 and thus, peptide titrations performed on K562 cells may indicate a lower peptide sensitivity than peptide titrations performed on T2 cells. In addition, T2 cells are TAP deficient, which leads to decreased endogenous peptide loading on HLA molecules at the cell surface and thus, exogenously pulsed peptides experience less competition for HLA binding. This may lead to higher density of pMHC complexes carrying the exogenously pulsed peptide at the cell surface and thus again, peptide titration assays may indicate a higher peptide sensitivity of a TCR when performed on T2 cells compared to K562 cells.

Decisively, T cells expressing the TCR constructs of the invention are also able to specifically recognize MAGEA4-positive tumor cells, e.g., A375 and H1703 cells.

Optionally, the TRA comprises a CDR1 having at least 60% sequence identity to SEQ ID NO: 2, a CDR2 having at least 71% sequence identity to SEQ ID NO: 3 and a CDR3 having at least 84%, preferably, 100% sequence identity to SEQ ID NO: 4. Optionally, the TRB comprises a CDR1 having at least 60% sequence identity to SEQ ID NO: 5, a CDR2 having at least 66% sequence identity to SEQ ID NO: 6 and a CDR3 having at least 88%, preferably, 100% sequence identity to SEQ ID NO: 7.

Optionally, individually or in combination, at most one amino acid in a CDR region differs (e.g., by substitution, deletion or insertion) from the defined sequences, e.g., in one, in two or in three CDR regions of one chain. Optionally, the TRA comprises a CDR1 having at least 80% sequence identity to SEQ ID NO: 2, a CDR2 having at least 85% sequence identity to SEQ ID NO: 3 and a CDR3 having at least 92%, preferably, 100% sequence identity to SEQ ID NO: 4. Optionally, the TRB comprises a CDR1 having at least 80% sequence identity to SEQ ID NO: 5, a CDR2 having at least 83% sequence identity to SEQ ID NO: 6 and a CDR3 having at least 94%, preferably, 100% sequence identity to SEQ ID NO: 7.

The TRA may comprise junction amino acids of SEQ ID NO: 10. The TRB may comprise junction amino acids of SEQ ID NO: 11

The TRA may comprise a variable region having at least 80%, optionally, at least 90% or 100% sequence identity to SEQ ID NO: 8. The TRB may comprise a variable region having at least 80%, optionally, at least 90% or 100% sequence identity to SEQ ID NO: 9.

A TCR construct comprising a TRA with a variable region of SEQ ID NO: 8 and a TRB with a variable region of SEQ ID NO: 9 has been shown to have particularly advantageous characteristics herein. It is also designated TCR127 or CTC-TCR127.

The variable region of the TRA of said TCR construct may be encoded by a nucleic acid having a sequence of SEQ ID NO: 12, and the variable region of the TRB of said TCR construct may be encoded by a nucleic acid having a sequence of SEQ ID NO: 13.

The TRA of the invention may comprise a TRAV26-1*02 sequence. The TRB of the invention may comprise a TRBV13*01 sequence.

In any of the TRA and/or TRB constructs of the invention, independently, in the CDR1, CDR2 and CDR3, there may be at most three, at most two or at most one amino acid deviating from the defined sequence specified herein, e.g., by substitution, deletion or insertion.

In any of the TRA and/or TRB constructs of the invention, independently, the CDR1, CDR2 and CDR3 may have at least 80%, at least 90% or 100% sequece identity to the recited sequences. Preferably, at least the CDR3 has 100% sequence identity to the recited defined CDR3. Typically, if the sequence is not identical, there is at most two, preferably, at most one amino acid exchange, deletion or insertion, typically, an amino acid exchange. The exchange may be a substitution, optionally, a conserved substitution, i.e., one amino acid of a particular type (e.g., polar, apolar, acidic, basic, aromatic) is exchanged against another amino acid of the same type. The exchange, deletion or substitution may lead to an increased affinity. Methods for affinity maturation are known in the art and further described below.

Optionally, the sequence identity to the recited CDR1 and CDR2 and CDR3 regions is 100% in the TRA or TRB, preferably, in both TRA and TRB of the TCR constructs of the invention.

A TCR alpha and/or beta chain construct of the invention may comprise all characteristics or domains corresponding to its native counterpart, i.e., a TCR alpha or beta chain, but this is not essential. Preferably, the TCR alpha and/or beta chain construct comprises at least a variable region, or a variable and a constant region, e.g., the variable and/or constant region having at least 60%, at least 70%, at least 80%, at least 90% or at least 95% sequence identity to a human variable or constant TCR region.

The TCR alpha chain constructs and/or the TCR beta chain constructs of the invention preferably comprise a constant region. For adoptive TCR-T cell therapy, it is preferred that the TCR construct comprises full length TCR alpha and beta chains comprising variable and constant regions, including transmembrane regions. The constant region may be a human constant region, a murine constant region or a chimeric constant region such as a minimally murine constant region. The TCR construct may be of essentially or exclusively human origin to minimize immunogenicity. To prevent pairing with endogenous TCR chains, the constructs of the invention may optionally contain one or more, e.g., 1-5, 1-10 or 1-20, preferably, 9 amino acid exchanges in comparison to a human sequence (Sommermeyer and Uckert, 2010).

To prevent pairing with endogenous TCR chains, the constant region of the TCR alpha and beta chain construct may be a murine constant region (Cohen et al., 2006), e.g, it may have at least 80% or at least 90% sequence identity to a murine constant region. TCRs with a murine constant region typrically have a stronger expression than with human constant regions.

Additional cysteines may alternatively or additionally be provided to enable the formation of an additional disulfide bond between the TCR chains (Cohen et al., 2007, Kuball et al., 2007). In addition, codon modification of the TCR sequence may be used to enhance the functional expression of the transgenic TCR (Scholten et al., 2006) and/or a peptide (e.g. P2A) can be applied to link both TCR chains to achieve a stoichiometric expression of both chains (Leisegang et al., 2008), also resulting in an enhanced functional expression of the transgenic TCR.

The construct may also be a chimeric antigen receptor, or part of it, wherein, e.g. a human TCR variable region may be linked to a different immunoglobulin constant domain, e.g. an IgG constant domain, and/or to an antibody domain capable of specifically binding to an antigen such as a MAGE antigen, e.g., MAGEA1, MAGEA3, MAGEA4 or MAGEA6, preferably, MAGEA4.

Single chain constructs (scTCR) are encompassed as well as heterodimeric TCR constructs. A scTCR can comprise a variable region of a first TCR chain construct (e.g., an alpha chain) and an entire (full-length) second TCR chain (e.g., a beta chain), or vice versa. Furthermore, the scTCR can optionally comprise one or more linkers which join the two or more polypeptides together. The linker can be, for instance, a peptide which joins together two single chains. Also provided is such a scTCR of the invention, which is fused to a cytokine, e.g., a human cytokine, such as IL-2, IL-7, IL-12 or IL-15.

To enable specific recognition of the epitope in the context of the MHC, in particular, for therapeutic purposes, the nucleic acid of the invention encodes one TCR alpha and one beta chain construct of a TCR construct of the invention, e.g., the TCR construct specific for the epitope of SEQ ID NO: 1, as described herein.

Typically, the nucleic acid sequences provided by the invention are codon-optimized for expression in human cells.

In the context of the invention, the nucleic acid can either be DNA or RNA. Preferably, it is DNA. The nucleic acid may, e.g., be a viral vector, or a non-viral vector, e.g., a transposon, a vector suitable for CRISPR/CAS-based, zinc finger nucleases-based or TALEN-based recombination, or a plasmid suitable for *in vitro* RNA transcription. The nucleic acid of the invention preferably is a vector. Suitable vectors include those designed for propagation and expansion, or for expression or both, such as plasmids and viruses. The vector may be an expression vector suitable for expression in a host cell selected from the group comprising a human T cell or a human T cell precursor, preferably, a human T cell such as CD8+ T cell and/or CD4+ T cell, typically, a CD8+ T cell. Said T cell may be a central-memory T cell, effector-memory T cell, effector T cell, stem cell memory T cell, stem cell-like T cell or naïve T cell, or a mixture of these.

The vector may be a viral vector, e.g. a retroviral, in particular gamma-retroviral or lentiviral vector. Examples of suitable expression vectors include the retroviral vector MP71 (Engels et al., 2003). The expression vector comprises regulatory sequences, such as transcription and translation initiation and termination codons, which are specific to the type of host cell (e.g., bacterium, fungus, plant, or animal) into which the vector is to be introduced and in which the expression of the nucleic acid of the invention shall be performed, typically, in the context of the invention, human CD8+ T cells. Furthermore, the vector of the invention may include one or more marker genes, which allow for selection of transduced or transfected hosts. The recombinant expression vector can comprise a native or, preferably, a heterologous promoter operably linked to the nucleotide sequence encoding the TCR construct of the invention, or to a nucleotide sequence which is complementary to or which hybridizes to the nucleotide sequence encoding the constructs of the invention. The selection of promoters includes, e.g., strong, weak, inducible, tissue-specific and developmental-specific promoters. The promoter can be a non-viral promoter or a viral promoter. Preferably, it is a heterologous promoter, i.e., a promoter not naturally linked to TCR in human T cells, such as long terminal repeat promoter, which is suitable for expression e.g. in human T cells. The inventive recombinant expression vectors can be designed for either transient expression, for stable expression, or for both. Also, the recombinant expression vectors can be made for constitutive expression or for inducible expression.

### Proteins

The present invention also provides a protein, i.e., an alpha or beta chain construct, or, preferably, a TCR construct comprising both alpha and beta chain constructs specific for an epitope of MAGEA4 in complex with a human MHC I, wherein the epitope has the sequence of SEQ ID NO: 1, the MHC I is HLA-A*01:01, and the TRA comprises a CDR3 having at least 84% sequence identity to SEQ ID NO: 4 and the TRB comprises a CDR3 having at least 88% sequence identity to SEQ ID NO: 7, as described herein. The protein is encoded by a nucleic acid of the invention.

The terms "capable of specifically binding" or "recognizing" or "specific for" a given epitope (herein, typically, the peptide of SEQ ID NO: 1 in the context of HLA-A1), as used herein, are synonymous, and mean that the TCR construct can specifically bind to and immunologically recognize said epitope, more preferably with high functional avidity. Preferably, a TCR may be considered to "be able of specifically binding" to an epitope if T cells expressing the TCR have an EC50 of at most 1000 nM, preferably, at most 100 nM, e.g., at most 10 nM. The EC50 value may be determined, as described above, using K562 cells. Alternatively, or additionally, a TCR may be considered to have "antigenic specificity" for an epitope if T cells expressing the TCR secrete at least twice as much IFNγ as the untransduced background level of IFNγ upon co-culture with target cells pulsed with a low concentration of the appropriate peptide. Such "specificity" as described above can - for example - be analyzed with an ELISA.

A high avidity is correlated with a high affinity - and with a high peptide sensitivity, e.g., with a half maximum IFN-γ release with a peptide concentration of 10⁻⁶ mol/L or less, preferably, 10⁻⁷ mol/L or less, preferably, 10⁻⁸ mol/L or less. Alternatively, affinity can be analyzed by methods well known to the skilled person, e.g. by BiaCore. A TCR affinity or T cell avidity of 100 nM or higher, more preferably 10 nM or higher is considered high affinity.

Based on the defined CDR3 and variable region sequences provided by the invention, it is possible to carry out affinity maturation of the TCR sequences (Chervin et al., 2008; Robbins et al., 2008). Non-synonymous nucleotide substitutions, which lead to amino acid exchanges in the CDR3 sequence, may lead to enhanced affinity of the TCR to target antigen. Furthermore, TCR sequence changes in other parts of the variable TRA and TRB regions may change affinity of the TCR to the peptide-MHC I complex. This may increase overall affinity of the TCR to the peptide-MHC, but harbors the risk of unspecific recognition and increased cross-reactivity (Linette et al., 2013). It is preferred that TCRs varying from the specific sequences provided retain exclusive specificity for the target antigen provided, i.e., that they are not cross-reactive, most importantly, that they do not have cross-reactivity for human self-peptides expressed on healthy cells. Potential cross-reactivity of TCRs can be tested against known self-peptides loaded on cells with the correct MHC allele (Morgan et al., 2013), e.g., as described herein. Accordingly, it is preferred that adoptive transfer of T cells expressing the TCR construct of the invention has no or significant negative effects on healthy tissue.

The TCR construct according to the invention can also be provided in the form of a multimeric complex, e.g., comprising at least two scTCR molecules, wherein said scTCR molecules are each fused to at least one biotin moiety, and wherein said scTCRs are interconnected by biotin-strepavidin interaction to allow the formation of said multimeric complex. Also provided are multimeric complexes of a higher order, comprising more than two, e.g., four, scTCR of the invention.

The TCR construct of the invention can be modified to comprise a detectable label, such as, for instance, a radioisotope, a fluorophore (e.g., fluorescein isothiocyanate (FITC), phycoerythrin (PE)), a tag, such as a HIS-tag, an enzyme (e.g., alkaline phosphatase, horseradish peroxidase), or particles (e.g., gold particles or magnetic particles).

### Host cells

The invention also provides a host cell comprising a nucleic acid and/or protein of the invention. The host cell can be a eukaryotic cell, e.g., plant, animal, fungi, or algae, or can be a prokaryotic cell, e.g., bacteria or protozoa. The host cell can be a cultured cell or a primary cell, i.e., isolated directly from an organism, e.g., a human. The host cell can be an adherent cell or a suspended cell, i.e., a cell that grows in suspension. For purposes of producing a recombinant TCR, polypeptide, or protein, the host cell preferably is a mammalian cell. Most preferably, the host cell is a human cell. The host cell can be of any cell type, can originate from any type of tissue, and can be of any developmental stage. For example, it can be a stem cell, such as a hematopoietic stem cell, or a hematopoietic pre-cursor cell. The host cell preferably is a peripheral blood leukocyte (PBL) or a peripheral blood mononuclear cell (PBMC). More preferably, the host cell is a T cell or T cell precursor, in particular, a human T cell, which may be isolated from PBMC. The T cell can be any T cell, such as a cultured T cell, e.g. a primary T cell, or a T cell from a cultured T cell line, or a T cell obtained from a mammal. Preferably, it is a T cell or T cell precursor from a human patient. The T cell can be obtained from numerous sources, such as blood, bone marrow, lymph node, the thymus, or other tissues or fluids. T cells can also be enriched for or purified. It can be, e.g., a tumor infiltrating cell (TIL), effector cell, central effector cell, memory T cell, naive T cell, and the like, preferably a central-memory T cell. Alternatively, the host cell may be another immune effector cell, e.g., a gamma-delta T cell, an NK cell, or a macrophage.

Preferably, in particular, in the context of therapy of a human, the T cell is a human T cell. The T cell preferably is a CD8+ cell (e.g., cytotoxic T cell) and/or CD4+ cell, in particular, a CD8+ cell. Preferably, the T cell is a T cell isolated from a human, e.g., a human patient, in particular, the patient who is to be treated, as this minimizes immune responses against the T cells after administration. Alternatively, the T cell may be from a third-party donor who may be related or unrelated to the patient. Such T cells may be genetically engineered, e.g., in multiple ways, for example, by knocking out of the endogenous TCR and/or MHC I . T cells may also be generated from autologous or third-party donor stem cells, wherein, optionally, the stem cells are not human embryonic stem cells.

T cells of the invention may express costimulatory molecules, such as CD8. Optionally, they are engineered to express CD8.

T cells of the invention may express a molecular enhancer element. Such an enhancer element may by a cytokine trap, dominant negative cytokine receptors, a molecular switch translating a negative into a positive T cell signal, a CAR-based construct improving the effector function of T cells, a miRNA, an siRNA or any other endogenous, or transgenic element or molecule improving T cell function e.g. in hematological or solid tumors. Function of inventive T cells may also be enhanced by exogenously adding active agents,

T cells of the invention may optionally express a safety element which allows their deletion in case an adverse effect is observed. For example, a truncated EGFR molecule that enables the depletion of transgenic T cells using an EGFR antibody or a safety switch inducing apoptosis of the therapeutic T cell upon administration of a specific molecule.

T cells may further express a detectable marker, e.g., useful for selection. The TCR of the invention can however also be directly used for selection, so an additional marker is not required.

Before transfer of the expression vector encoding the TCR construct of the invention (e.g., transfection or transduction), the T cells may be expanded. They can alternatively or additionally be expanded after transfer of the expression vector encoding the TCR construct of the invention.

Preferably, the host cell is a human T cell, e.g., CD8+ T cell, comprising a nucleic acid of the invention which is an expression vector, wherein the nucleic acid encoding the TRA and/or TRB is operably linked to a heterologous promotor, wherein the host cell expresses a TCR construct of the invention.

### Pharmaceutical compositions

The invention also provides a pharmaceutical composition comprising
a) a nucleic acid of the invention encoding a TCR construct capable of specifically binding to its respective epitope in the context of the respective MHC I (i.e., a TCR construct of the invention); or
b) a protein of the invention comprising a TCR construct capable of specifically binding to its respective epitope in the context of the respective MHC I (i.e., a TCR construct of the invention); or
c) a host cell of the invention expressing a TCR construct capable of specifically binding to its respective epitope in the context of the respective MHC I (i.e., a TCR construct of the invention).

The pharmaceutical composition of the invention is for use in the treatment of a patient expressing HLA-A1 and having a MAGEA4-positive cancer, e.g., a tumor selected from the group comprising melanoma, gastric cancer, head and neck cancer, lung cancer, breast cancer, ovarian cancer, bladder cancer, and esophageal cancer.

The invention also provides a method for treating of a patient expressing HLA-A1 and having a MAGEA4-positive cancer, e.g., a cancer selected from the group comprising melanoma, gastric cancer, head and neck cancer, lung cancer, breast cancer, ovarian cancer, bladder cancer, and esophageal cancer, comprising administering an effective amount of the pharmaceutical composition of the invention to said patient.

The patient expresses HLA-A1, e.g., preferably, HLA-A*01:01. Average HLA (MHC I) distribution in different populations can be found, e.g., under http://allelefrequencies.net/. Before treatment, it should be known or tested if a patient expresses the respective HLA.

Accordingly, the kit or pharmaceutical composition may comprise human CD8+ T cells comprising a nucleic acid encoding a TCR construct of the invention, e.g., recognizing the epitope of SEQ ID NO: 1 in HLA-A1 context, as defined herein. The TCR construct may be expressed from the nucleic acid under the control of a heterologous promotor.

Adoptive T cell therapy (option c) is a preferred option for treatment throughout the present application, wherein the host cell is a T cell, preferably, a human CD8+ T cell. Said host cell typically comprises a nucleic acid encoding the TCR construct under the control of a heterologous promotor.

However, gene therapy with a nucleic acid of the invention is also possible (option a), wherein, e.g., a lentiviral vector may be used.

A TCR construct of the invention in protein form (option b) may also be used in therapy, e.g., for targeting a toxin to which it is linked to a cancer, or for targeting a vesicle, e.g., a liposome or a bacterial minicell to a cancer, which may comprise a therapeutic agent such as a toxin. A TCR construct of the invention in protein form may also be used for targeting a diagnostic agent to a cancer. The composition may thus also be a diagnostic composition.

The pharmaceutical or diagnostic compositions of the invention typically are for intravenous administration. They may further comprise pharmaceutically acceptable carriers such as buffers, e.g., physiological saline or PBS. They may further comprise exciptients, e.g., stabilizers such as SPGA, carbohydrates (e.g. sorbitol, mannitol, starch, sucrose, glucose, dextran), proteins such as albumin or casein, or protein containing agents such as bovine serum or skimmed milk.

T cells are typically administered to a patient in a concentration of 1 x 10⁵ - 5 x 10⁹ cells per kg. About 1 x 10⁶ - 5 x 10¹¹ cells, e.g., 1 x 10⁸ - 2 x 10¹⁰ cells may be administered to a patient as a single dose, i.v.. These parameters may be adapted by the medical practioner, depending, e.g., on the patients age, sex, body weight and medical condition. Protocols disclosed, e.g., in Doran et al., 2019 may be adapted to use the host cells of the present invention.

Typically, before administration of T cells, the patients are pre-conditioned, e.g., by administration of fludarabine and cyclophosphamide (e.g., infusions of 30 mg/m² fludarabine and 500 mg/m² cyclophosphamide), for example, on days 5, 4 and 3 prior to administration of T cells.

The patient is a mammalian patient. The patient may be a mouse expressing the required HLA, but, preferably, the patient is a human patient.

The T cell expressing its respective TCR construct may be contained in a kit, wherein, the T cells are stored, e.g., in a pharmaceutically acceptable buffer. Such a kit may comprise additional agents, e.g., for pre-conditioning the patient for T cell therapy, e.g., with fludarabine and cyclophosphamide. It may also comprise other anti-cancer agents. For example, other anti-cancer agents may be TCR-engineered T cells expressing TCRs specific for other antigens expressed in the MAGEA4-positive tumor (e.g. antigens such as EBV LMP2A, MAGEA1), checkpoint inhibitors or other immunotherapies as well as antibodies, small molecule inhibitors or other types of reagents. The components of a kit of the invention may be formulated for simultaneous administration or for administration in any sequence. The components may also be for repeated administration. Tran et al., 2014 describe a possible regimen of administration. Alternatively, they may be mixed before administration and administered together. Alternatively, the T cells each expressing its respective TCR construct may be contained in a single pharmaceutical composition. The same applies for nucleic acids or proteins of the invention.

One preferred medicinal use of the invention is immune therapy, preferably adoptive T cell therapy. The product and methods of the invention are particularly useful in the context of adoptive T cell therapy. The administration of the compounds of the invention can for example involve the administration, e.g., infusion of T cells of the invention into said patient. Preferably, such T cells are autologous T cells of the patient which were *in vitro-*engineered to express a nucleic acid of the present invention.

Alternatively, the patient may also be administered a nucleic acid of the invention, in particularly, an expression vector, for *in vivo* transduction of T cells.

Protein TCR constructs of the invention may also, e.g., be used for diagnostic purposes to find out if a subject expresses the respective protein, and, in particular, if the epitope recognized by the TCR construct in the context of the MHC I is presented. To this end, such constructs are preferably labelled to facilitate detection. Preferably, a patient presenting such an epitope on the respective MHC Ion tumor cells is treated by an adoptive T cell therapy of the invention.

The invention also relates to a method of preparing a host cell of the invention, comprising introducing an expression vector encoding a TCR construct of the invention into a suitable host cell, preferably, a human CD8+ T cell isolated from a patient.

### Vaccines

In one embodiment, the invention provides a pharmaceutical composition, in particular, a vaccine composition, comprising a peptide comprising SEQ ID NO: 1, SEQ ID NO: 18 or SEQ ID NO: 19. The peptide is capable of being presented on HLA-A*01-01, and comprises at most 11, preferably, at most 10 , or at most 9 consecutive amino acids identical to the sequence of amino acids ocurring in MAGEA4 (SEQ ID NO: 14). Preferably, the peptide consists of SEQ ID NO: 1, SEQ ID NO: 18 or SEQ ID NO: 19. SEQ ID NO: 1 was found to be recognized with the highest affinity, and is thus preferred. Alternatively, said pharmaceutical composition, or vaccine composition, comprises a nucleic acid (e.g., RNA) encoding said peptide.

The peptide in complex with HLA-A*01:01 is specifically recognized by TCR127 as defined herein.

Peptide vaccinations are well known in the state of the art. For example, peptide vaccines may be for use in administration to a subject in combination with an adjuvant, such as an aluminium salt, e.g., aluminium phosphate or aluminiom hydroxide, squalene, e.g., MF59, liposomes, e.g., QS21 or monophosphoryl lipid A.

Nucleic acids encoding a peptide comprising an epitope, as defined herein, may also be used, e.g., in combination with a suitable adjuvant such as liposomes or CpG nucleotides.

Any of said vaccine pharmaceutical compositions may be for use in vaccination against a MAGEA4-positive cancer, e.g., a cancer selected from the group comprising melanoma, gastric cancer, head and neck cancer, lung cancer, breast cancer, ovarian cancer, bladder cancer, and esophageal cancer. The vaccination may be preventive vaccination, i.e., a vaccination provided to a subject not yet having the cancer, with the intent of reducing the risk for the subject to develop the cancer, i.e., in case of a genetic predisposition or exposition to predisposing conditions, e.g., mutagens. The subject may belong to a risk group for the disease, wherein the subject does not have the disease (yet). Alternatively, the vaccination may be therapeutic vaccination. A patient having a MAGEA4-positive cancer may be treated with a therapeutic vaccination.

Advantageously, the HLA of the subject or patient is tested or known, and the vaccine is administered to a patient who is capable of presenting the epitope of SEQ ID NO: 1 on HLA-A1, e.g., HLA-A*01:01.

The present invention is further illustrated in the following examples with reference to the accompanying figures and sequences, nevertheless, without being limited thereto. For the purposes of the present invention, all references as cited herein are incorporated by reference in their entirety.

### Legends

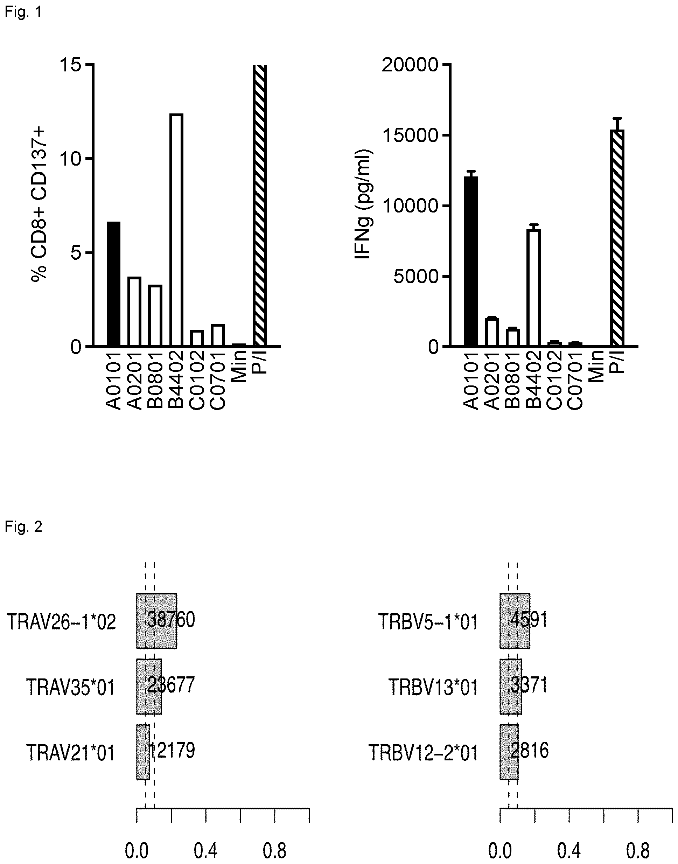

**Figure 1****.** Screening for epitope-HLA-specific T cells using a HLA cell library, as described in WO 2016146618 A1. Peripheral blood mononuclear cells (PBMCs comprising T cells) that had been stimulated repeatedly with autologous dendritic cells expressing the antigens MAGEA3, MAGEA4 and MAGEA6 were cocultured with antigen-transfected K562 cells of the HLA cell library, which were matched to all MHC class I alleles of the original PBMC donor. Reactivity of T cells to antigen-HLA-expressing K562 cells was determined by flow cytometric analysis of the CD137 activation marker upregulated on responding T cells as well as by cytokine release (IFNγ) to the supernatant measured by ELISA. Shown are means of duplicates +/- SEM. T cells without target cells and peptide (Min) and T cells unspecifically activated with PMA/ionomycin (P/I) served as controls.
**Figure 2****.** Deep-sequencing-based analysis of TCR gene sequences and frequencies of MAGEA3/MAGEA4/MAGEA6 and HLA-A*01:01-reactive T cells. Based on CD8 and CD137 expression, MAGEA3/ MAGEA4/MAGEA6 and HLA-A*01:01-reactive T cells were sorted and RACE-PCRs were performed to generate libraries suitable for Illumina HiSeq sequencing. Libraries were analyzed for frequencies of TCR alpha (TRAV) and TCR beta (TRBV) chains. Depicted are frequencies (fraction of reads) of the top three enriched TCR alpha (left) and TCR beta chains (right) and absolute number of reads, respectively. Dashed lines indicate fraction of reads of 0.05 and 0.1 (=frequencies of 5% and 10%) among all TRAV and TRBV reads.

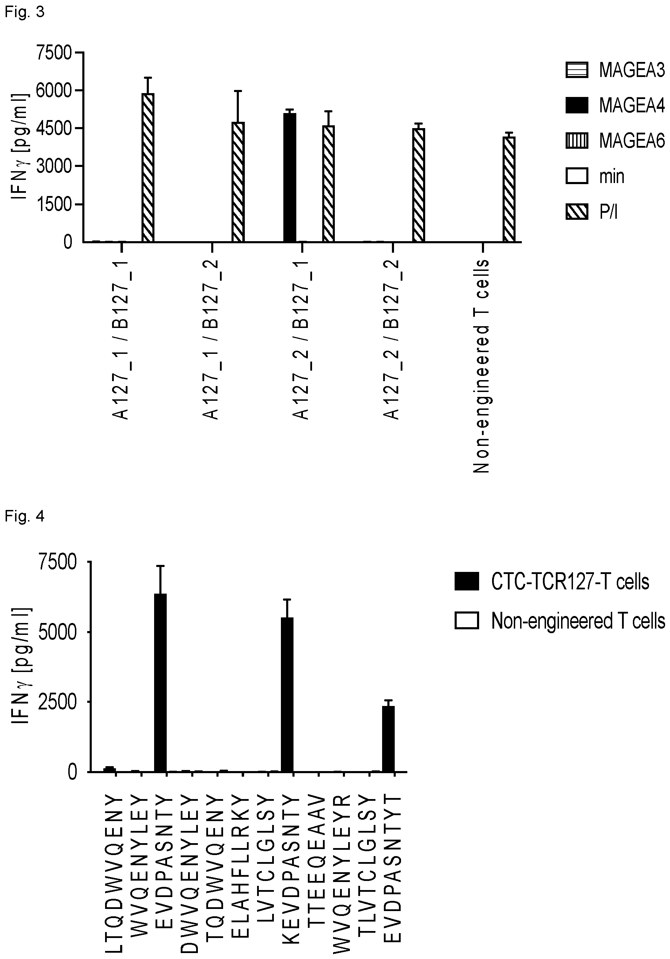

**Figure 3****.** Enriched TCR alpha (TRAV) and beta (TRBV) chains were expressed in different combinations and tested for functional specificity. Two alpha and two beta chains that were found to be enriched above 10% were synthesized, fused to murine constant TCR regions, and cloned into the MP71 retroviral vector. Retroviral particles were generated using a 293T cell-based transfection method and fresh PBMCs were transduced with the four different alpha and beta chain combinations. Resulting TCR-T cells were cocultured with K562-HLA-A*01:01 cells expressing the different target MAGE antigens (MAGEA3, MAGEA4 and MAGEA6) to determine which alpha and beta chain combination forms the functional TCR that was responsible for the signal in the initial HLA cell library screen. IFNγ release of TCR-T cells was measured by ELISA. Shown are means of duplicates +/-SEM. min, K562-A*0101 target cells without TCR-T cells; P/I, unspecific activation of bulk T cells by PMA/ionomycin.
**Figure 4****.** Epitope mapping for CTC-TCR127. CTC-TCR127-T cells were cocultured with K562-A*01:01 cells that were pulsed with different 9- and 10-mer peptides predicted to bind to HLA-A*01:01. Reactivity of CTC-TCR127-T cells was assessed by measuring IFNγ release to the supernatant via ELISA. Shown are means of duplicates +/- SEM. The peptides have SEQ ID NOs: 20, 21, 1, 22, 23, 24, 25, 18, 26, 27, 17 and 19, from left to right.
**Figure 5****.** Identification of the cognate peptide of CTC-TCR127. The three recognized MAGEA4 peptides (>95% purity) were pulsed at 10⁻⁸ M on K562-A*01:01 cells and cocultured for 18 h with CTC-TCR127-T cells. IFNγ release to the supernatant was measured by ELISA. Shown are means of duplicates +/- SEM. K562-A*0101 target cells without peptide served as negative control. The peptides have SEQ ID NOs: 1, 18 and 19.
**Figure 6****.** To determine the affinity of CTC-TCR127, 1x10⁵ T cells transduced with CTC-TCR127 were cocultured with 5x10⁴ peptide-loaded K562-A*0101 cells and IFNγ release was measured by ELISA after 18 hours. The results of two donors were pooled and normalized as percentage of max. The depicted EC50 value was calculated by non-linear regression.

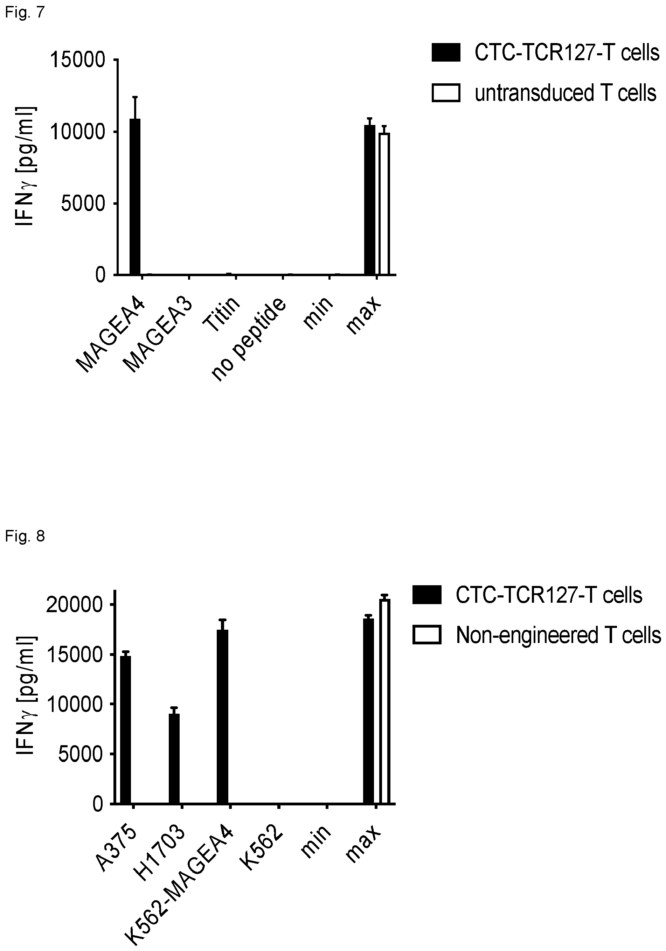

**Figure 7****.** Cross-reactivity of CTC-TCR127 to sequence similar epitopes. CTC-TCR127-T cells were cocultured with K562-A*01:01 cells that were pulsed with either the cognate MAGEA4 peptide EVDPASNTY (SEQ ID NO: 1) or the sequence similar peptides of MAGEA3 (EVDPIGHLY, SEQ ID NO: 15) and titin (ESDPIVAQY, SEQ ID NO: 16). IFNγ release to the supernatant was measured by ELISA after 18 hours of coculture. Shown are means of duplicates +/- SEM of one representative T cell donor. min, CTC-TCR127-T cells without target cells and peptide; max, T cells activated with PMA/ionomycin.
**Figure 8****.** Tumor cell recognition by CTC-TCR127-T cells. CTC-TCR127-T cells were tested for recognition of naturally HLA-A*01:01 and MAGEA4-positive tumor cell lines (A375, H1703) and the HLA-A*01:01 and MAGEA4-engineered cell line K562 via a coculture assay. IFNγ release by responding CTC-TCR127-T cells to the supernatant was measured by ELISA after 18 hours. Shown are means of duplicates +/- SEM of one representative T cell donor. min, CTC-TCR127-T cells without target cells; max, T cells unspecifically activated with PMA/ionomycin.

**Sequences**

| | | |
|---|---|---|
| SEQ ID NO: 1 | MAGEA4 epitope presented on HLA-A*01:01 | EVDPASNTY |
| SEQ ID NO: 2 | TRA CDR1, aa | SIFNT |
| SEQ ID NO: 3 | TRA CDR2, aa | LYKAGEL |
| SEQ ID NO: 4 | TRA CDR3, aa | AGQLWGGSNYKLT |
| SEQ ID NO: 5 | TRB CDR1, aa | SGHRS |
| SEQ ID NO: 6 | TRB CDR2, aa | YFSETQ |
| SEQ ID NO: 7 | TRB CDR3, aa | ASSLAGTGAHFITNEKLF |
| SEQ ID NO: 8 | TRA variable region, aa | |
| GQQLNQSPQSMFIQEGEDVSMNCTSSSIFNTWLWYKQEPGEGPVLLIALYKAGELTSNGRLTAQFG ITRKDSFLNISASIPSDVGIYFCAGQ | | |
| SEQ ID NO: 9 | TRB variable region, aa | |
| KAGVTQTPRYLIKTRGQQVTLSCSPISGHRSVSWYQQTPGQGLQFLFEYFSETQRNKGNFPGRFS GRQFSNSRSEMNVSTLELGDSALYLCASSL | | |
| SEQ ID NO: 10 | TRA junction aa | CAGQLWGGSNYKLTF |
| SEQ ID NO: 11 | TRB junction aa | CASSLAGTGAHFITNEKLFF |
| SEQ ID NO: 12 | TRA variable region, codon-optimized na | |
| GGCCAGCAGCTGAATCAGAGCCCACAGAGCATGTTCATCCAAGAAGGCGAGGACGTTTCCATG AACTGCACCAGCAGCAGCATCTTCAACACCTGGCTGTGGTACAAGCAAGAGCCTGGCGAAGGA CCCGTGCTGCTGATTGCCTTGTATAAGGCCGGCGAGCTGACCAGCAACGGCAGACTGACAGC CCAGTTCGGCATTACCCGGAAGGACAGCTTCCTGAACATCTCCGCCAGCATTCCCAGCGACGT GGGCATCTATTTTTGTGCCGGACAG | | |
| SEQ ID NO: 13 | TRB variable region, codon-optimized na | |
| AAAGCTGGCGTGACCCAGACACCTAGATACCTGATCAAGACCAGAGGCCAGCAAGTGACCCTG AGCTGCTCTCCTATCAGCGGCCACAGAAGCGTGTCCTGGTATCAGCAGACACCTGGACAGGGC CTGCAGTTCCTGTTCGAGTACTTCAGCGAGACACAGCGGAACAAGGGCAACTTCCCCGGCAGA TTTTCCGGCAGACAGTTCAGCAACAGCCGCAGCGAGATGAACGTGTCCACACTGGAACTGGGC GACAGCGCCCTGTATCTGTGTGCCTCTTCTCTT | | |
| SEQ ID NO: 14 | MAGEA4, aa | |
| | MSSEQKSQHCKPEEGVEAQEEALGLVGAQAPTTEEQEAAVSSSSPLVPGTLEEVPAAESAGPPQS PQGASALPTTISFTCWRQPNEGSSSQEEEGPSTSPDAESLFREALSNKVDELAHFLLRKYRAKELV TKAEMLERVIKNYKRCFPVIFGKASESLKMIFGIDVKEVDPASNTYTLVTCLGLSYDGLLGNNQIFPKT GLLIIVLGTIAMEGDSASEEEIWEELGVMGVYDGREHTVYGEPRKLLTQDWVQENYLEYRQVPGSN PARYEFLWGPRALAETSYVKVLEHVVRVNARVRIAYPSLREAALLEEEEGV | |
| SEQ ID NO: 15 | MAGEA3 epitope | EVDPIGHLY |
| SEQ ID NO: 16 | titin epitope | ESDPIVAQY |
| SEQ ID NO: 17 | MAGEA4 peptide | TLVTCLGLSY |
| SEQ ID NO: 18 | 10mer MAGEA4 epitope pres. on HLA-A*01:01 | KEVDPASNTY |
| SEQ ID NO: 19 | 10mer MAGEA4 epitope pres. on HLA-A*01:01 | EVDPASNTYT |
| SEQ ID NO: 20 | MAGEA4 peptide | LTQDWVQENY |
| SEQ ID NO: 21 | MAGEA4 peptide | WVQENYLEY |
| SEQ ID NO: 22 | MAGEA4 peptide | DWVQENYLEY |
| SEQ ID NO: 23 | MAGEA4 peptide | TQDWVQENY |
| SEQ ID NO: 24 | MAGEA4 peptide | ELAHFLLRKY |
| SEQ ID NO: 25 | MAGEA4 peptide | LVTCLGLSY |
| SEQ ID NO: 26 | MAGEA4 peptide | TTEEQEAAV |
| SEQ ID NO: 27 | MAGEA4 peptide | WVQENYLEYR |

### Examples

### Generation of MAGEA4-specific TCR constructs and transgenic T cells, and identification of epitopes

Using RNAseq data of solid tumors, the inventors found that the CTA antigens MAGEA3, -A4 and - A6 are expressed in several tumor entities while their expression is absent in healthy adult tissue (GTEX database) and only found in testis, which is an immune privileged tissue.

The inventors generated *in vitro-*transcribed RNA (ivtRNA) for all three antigens using the mMessage mMachine kit (Thermo Fisher Scientific, Dreieich, Germany) followed by poly-adenylation of ivtRNA using the poly(A) tailing kit from Thermo Fisher Scientific. Peripheral blood mononuclear cells (PBMC) were isolated using Ficoll gradient centrifugation of fresh blood samples. Mature dendritic cells (mDCs) were generated from PBMCs using a modified plate-adherent monocyte protocol (Spranger et al, 2010). mDCs were transfected with antigen ivtRNA using electroporation. At this step, the endogenous expression of antigens in DCs is key to enable processing and presentation of immunodominant epitopes via the best-suited HLA alleles at the cell surface. Only such true antigenic epitope/HLA complexes can be efficiently targeted by T cells. Next, naïve T cells were isolated from PBMCs of the same blood donor as the mDCs (autologous setting) using the naïve T cell sorting kit from Stemcell Technologies (Cologne, Germany).

Stimulation of naïve T cells with antigen-expressing mDCs was carried out in 24-well plates, supplementing low-dose IL2 and IL7 three times per week. After 10-14 days, expanded T cells were restimulated with antigen-expressing DCs and cultured for another 10-14 days.

Next, we used single HLA-expressing K562 cells from our HLA cell library (WO 2016146618 A1) resembling all six HLA alleles of the original T cell donor and transfected them with the three MAGE antigens that had been used for stimulating the T cells. After 20 h, culture supernatant was analyzed by ELISA to assess IFNγ release by T cells, and as a second read-out, T cells were stained for CD137 activation marker expression and analyzed using flow cytometry.

Figure 1 shows reactivity of T cells to the different MAGE/HLA combinations measured by CD137 expression and IFNγ release. Significant responses were seen with HLA-A*01:01 cells and HLA-B*44:02 cells. The T cell population specific for the MAGEA4-expressing K562-HLA-A*01:01 cells contains the T cells from which the TCR of the invention, CTC-TCR127 was later isolated.

**Identification of reactive T cells:** T cells responding to a specific antigen-HLA combination were sorted based upon CD137 expression using FACS. RNA from sorted T cells was isolated using an RNeasy micro kit from Qiagen (Hilden, Germany). SMARTer RACE PCR for Illumina HiSeq from Clontech (Takara, Saint-Germain-en-Laye, France) was used to generate RACE-PCR products. PCR products were pooled in libraries of 2-5 samples and sequenced by Novogene (Beijing, China) using Illumina HiSeq machines.

**Bioinformatic analysis** was performed to identify the TCR gene sequences and the frequencies of TCR alpha and beta chains that were enriched in the T cell population that was reactive to the MAGE antigens in combination with HLA-A*01:01 (Figure 2). The two most frequent TCR alpha and beta chains, respectively, were picked for further analysis.

**CTC-TCR127 (also designated TCR127) was discovered** by expressing different combinations of the two most enriched TCR alpha and beta chains in fresh T cells and testing them for recognition of K562-A*0101 cells expressing either MAGE-A3, -A4 or -A6. T cells transduced with the TRAV35*01 and the TRBV5-1*01 chains specifically recognized K562-A0101 cells expressing MAGEA4 (Figure 3). As previous experiments showed repeatedly, only the combination of the correct chains results in a functional TCR, while T cells carrying one functional and one irrelevant TCR chain do not recognize antigen-positive tumor cells at all. Furthermore, CTC-TCR127 exclusively recognized MAGEA4, although MAGEA3 and MAGEA6 contain large stretches of similar sequences, thus showing the high specificity of CTC-TCR127.

**To identify the cognate epitope,** CTC-TCR127-T cells were tested against a panel of different 9- and 10-mer epitopes of MAGEA4 that were predicted by netMHCpan4.0 (Department of Health Technology, Lyngby, Denmark) to bind to HLA-A*01:01. For this, K562-A0101 cells were pulsed with 10-6 mol/L of the peptides and cocultured with CTC-TCR127-T cells. Three MAGEA4 peptides were recognized by the CTC-TCR127-T cells with EVDPASNTY (SEQ ID NO: 1) showing the highest IFNγ release (Figure 4). All three peptides contain the same core sequence since KEVDPASNTY (SEQ ID NO: 18) and EVDPASNTYT (SEQ ID NO: 19) are corresponding 10mers to the 9mer EVDPASNTY. CTC-TCR127 showed high specificity to the three peptides and no background recognition of the other peptides or K562-A0101 without pulsed peptide.

To identify the cognate epitope of CTC-TCR127, the three recognized epitopes were synthesized at high purity (>95%) and pulsed at the concentration of 10⁻⁸ mol/L on K562-A*0101 cells, which were further cocultured with CTC-TCR127-T cells. At this concentration, CTC-TCR127-T cells only retained a strong functional recognition for K562-A*0101 cells pulsed with EVDPASNTY (SEQ ID NO: 1), while IFNγ release as a response to KEVDPASNTY (SEQ ID NO: 18) and EVDPASNTYT (SEQ ID NO: 19) was almost abrogated, indicating that the peptide of SEQ ID NO: 1 is the cognate MAGEA4 epitope of CTC-TCR127 (Figure 5).

To determine the **functional avidity of TCR127,** results from two donors were pooled and normalized as percentage of maximum IFNγ. To calculate the EC50 value, non-linear regression was used to create a dose-response curve (Figure 6). TCR127 showed an EC50 of 7.6 nM. It should be noted that K562 cells, in contrast to T2 cells usually used for HLA-A*02:01 peptides, do not express the co-stimulatory molecule CD80 and HLA molecules on the surface are already loaded with endogenous peptides. Thus, peptide titration curves generated on T2 and K562 cells cannot be compared directly, as T2 cells might indicate a higher sensitivity of TCRs to peptides. In the context of the present invention, the EC50 value is preferably determined based on a titration of the peptide of SEQ ID NO: 1 on K562 cells expressing HLA-A*01:01, wherein 5x10⁴ K562 cells are used and 1x10⁵ TCR-transduced T cells are used, and IFNγ release after 18 hour incubation is determined by ELISA, e.g., as carried out for the experiment underlying Fig. 6. The K562 cells express HLA-A*01:01. The T cells have a transduction rate of at least 50%, e.g., about 65-80% of the T cells used express the transgenic TCR. The cell viability is high, e.g., at least 60%, preferably, at least 80%.

Cross-reactivity to sequence similar antigens or peptides is a major concern of TCR-T cell therapy as previous reports from fatal toxicities in the clinic have shown (Spranger et al., 2010). For example, a MAGEA3-specific HLA-A*01:01-restricted TCR was found to be cross-reactive to a protein called titin that is expressed in the heart (Cameron et al., 2013). Interestingly, the cognate peptide of CTC-TCR127 (EVDPASNTY, SEQ ID NO: 1) showed some sequence similarity with the MAGEA3 peptide (EVDPIGHLY, SEQ ID NO: 15) and the titin (ESDPIVAQY, SEQ ID NO: 16) peptides that were characterized in these studies. To exclude that CTC-TCR127 may be cross-reactive to these epitopes, CTC-TCR127-T cells were cocultured with K562-A*0101 cells loaded with either the MAGEA3 peptide EVDPIGHLY (SEQ ID NO: 15) or the titin peptide ESDPIVAQY (SEQ ID NO: 16). TCR127 showed no recognition of the MAGEA3 peptide or titin peptide (Figure 7).

For clinical applications, a strong reactivity of CTC-TCR127-T cells against tumor cells is crucial. To assess the potential of CTC-TCR127 to recognize tumor cells, we cocultured CTC-TCR127-T cells with the MAGEA4-positive tumor cell lines A375 (a melanoma cell line) and H1703 (a non-small cell lung cancer cell line) both naturally expressing HLA-A*01:01. CTC-TCR127-T cells showed a robust recognition of both cell lines as assessed by IFNγ, which was used as surrogate marker for antigen-specific tumor cell killing. Recognition was comparable with that of K562-A*0101 cells, that were engineered to express HLA-A*01:01 and MAGEA4 (Figure 7).

### Conclusion

Using the patented TCR and epitope discovery platform, we were able to generate a highly effective TCR directed against the melanoma-associated antigen 4-positive (MAGEA4) restricted to HLA-A*01:01. CTC-TCR127-T cells demonstrated specific recognition of its cognate peptide and MAGEA4-positive tumor cell lines, while triggering no response to sequence similar peptides of MAGEA3 and titin or a MAGEA4-negative tumor cell lines. To our knowledge, this is the first MAGEA4-specific TCR for the HLA-A*01:01 patient population.

MAGEA4 is a highly promising target for TCR-T cell therapy of solid tumors as it is a widely expressed shared antigen found in several solid tumor entities. It is a safe target due to its restricted expression in tumor cells and testis tissue, which cannot be accessed by T cells. Recently, Immatics and Adaptimmune published promising clinical phase I data for TCR-T cell therapy trials targeting MAGEA4 (Immatics, 2021; Adptimmune, 2020; Sanderson, 2019; WO 2021023658 A1; WO 2017174824 A1). Medigene and Bluebird published another MAGEA4-TCR (Davari et al., 2021; EP 3714941 A1). However, these TCRs are restricted to patients carrying the HLA-A*02:01 allele. CTC-TCR127 is a promising candidate for TCR-T cell therapy of patients that do not express HLA-A*02:01 unlocking a large market that is currently not addressable.

### References

Abelin, J. G. et al., 2017. Immunity 46, 315-326.
Adaptimmune, 2020. Adaptimmune - Corporate Deck December 2020.
Adaptimmune, 2021. Adaptimmune press release https://d1io3yog0oux5.cloudfront.net/_fe704720d698f40777802d4ccedb64ac/adaptimmune/news/20 20-05-29_SPEAR_T_cells_Targeting_MAGE_A4_Demonstrate_New_172.pdf.
Cameron, B. J. et al., 2013. Sci Transl Med 5, 197ra103.
Chervin et al., 2008. Journal of Immunological Methods 339: 175-184.
Cho et al., 2018. British Journal of Cancer 118: 534-545.
Cohen et al., 2006. Cancer Research 66: 8878-8886.
Cohen et al., 2007. Cancer Research 67: 3898-3903.
Danska et al., 1990. The Journal of Experimental Medicine 172: 27-33.
Davari, K. et al., 2021. J Immunother Cancer 9, e002035.
Doran et al., 2019. Journal of Clinical Oncology doi: 10.1200/JCO.18.02424. [Epub ahead of print].
Engels et al., 2003. Human Gene Therapy 14: 1155-1168.
Garcia and Adams, 2005. Cell 122: 333-336.
Gotter, J. et al., 2004. J Exp Medicine 199, 155-166.
Hart et al., 2008. Gene Therapy 15: 625-631.
Immatics, 2021. Immatics - Corporate Presentation 2021.
Immatics, 2021. Immatics press relase https://ml-eu.globenewswire.com/Resource/Download/147fd094-fl5a-4e5e-b454-83c12f4e1cdc.
Ishihara, M. et al., 2020. Bmc Cancer 20, 606.
lura, K. et al., 2017. Virchows Arch 471, 383-392.
Juretic, A. et al., 2003. Lancet Oncol 4, 104-109.
Jurgens et al., 2006. Journal of Clinical Immunology 26: 22-32.
Kobayashi, T. et al., 2003. Tissue Antigens 62, 426-432.
Kuball et al., 2007. Blood 109: 2331-2338.
Leisegang et al., 2008. Journal of Molecular Medicine. 86: 573-583.
Linette et al., 2013. Blood 122: 863-871.
Lorenz et al., 2018. Human Gene Therapy 28: 1158-1168.
Morgan et al., 2013. Journal of Immunotherapy 36: 133-151.
Obenaus, M. et al., 2015. Nature biotechnology 33, 402-407.
Orentas et al., 2001. Clinical Immunology 98: 220-228.
Ottaviani, S. et al., 2006. Cancer Immunol Immunother 55, 867-872.
Ritz, D. et al., 2018. Proteomics 18, 1700246.
Robbins et al., 2008. Journal of Immunology 180: 6116-6131.
Sanderson, J. P. et al., 2019. Oncoimmunology 9, 1682381.
Scholten et al., 2006. Clinical Immunology 119: 135-145.
Simpson et al., 2011. Proceedings of the National Academy of Sciences of the USA 108: 21176-21181.
Sommermeyer and Uckert, 2010, Journal of Immunology 184: 6223-6231.
Spranger, S. et al., 2010. J Immunol 185, 738-747.
Tran et al., 2014. Science 344: 641-645.
Yang et al., 2011. Clinical and Developmental Immunology, Article ID 716926
Zheng et al., 2015. Cancer Immunology Research 3: 1138-1147.
EP 3714941 A1
WO 2016146618 A1 - Lorenz, Uckert, Ellinger & Schendel.
WO 2021023658 A1
WO 2017174824 A1
WO 2011/039508 A2
WO 2015/022520 A1

## Claims

1. A nucleic acid encoding a TCR alpha chain construct (TRA) and/or a TCR beta chain construct (TRB) of a TCR construct specific for an epitope of MAGEA4 in complex with a human MHC I, wherein the MHC I is HLA-A*01:01.

2. A nucleic acid of claim 1, wherein the epitope has the sequence of SEQ ID NO: 1, and the TRA comprises a CDR3 having at least 84% sequence identity to SEQ ID NO: 4 and/or the TRB comprises a CDR3 having at least 88% sequence identity to SEQ ID NO: 7.

3. A nucleic acid of any of the preceding claims, wherein the TRA comprises a CDR1 having at least 60% sequence identity to SEQ ID NO: 2, a CDR2 having at least 71% sequence identity to SEQ ID NO: 3 and a CDR3 having at least 84%, preferably, 100% sequence identity to SEQ ID NO: 4, and/or wherein the TRB comprises a CDR1 having at least 60% sequence identity to SEQ ID NO: 5, a CDR2 having at least 66% sequence identity to SEQ ID NO: 6 and a CDR3 having at least 88%, preferably, 100% sequence identity to SEQ ID NO: 7.

4. A nucleic acid of any of the preceding claims, wherein the TRA comprises a variable region having at least 90% sequence identity to SEQ ID NO: 8 and/or the TRB comprises a variable region having at least 90% sequence identity to SEQ ID NO: 9.

5. The nucleic acid of any of the preceding claims, wherein the sequence identity to the recited CDR3 regions is at least 92%,
wherein, preferably, the sequence identity to the recited CDR1 and CDR2 and CDR3 regions is 100%.

6. The nucleic acid of any of the preceding claims, wherein the TCR alpha chain construct and/or the TCR beta chain construct further comprise a constant region selected from the group comprising a human constant region, a murine constant region or a chimeric constant region.

7. The nucleic acid of any of the preceding claims, encoding at least one TCR alpha and beta chain construct of the TCR construct.

8. The nucleic acid of any of the preceding claims, wherein the nucleic acid is selected from the group comprising a viral vector, a transposon, a vector suitable for CRISPR/CAS based recombination or a plasmid suitable *for in vitro* RNA transcription.

9. A protein encoded by the nucleic acid of any of the preceding claims.

10. A host cell comprising a nucleic acid and/or a protein of any of the preceding claims, wherein the host cell preferably is a human T cell.

11. A pharmaceutical composition comprising
a) a nucleic acid of any of claims 1-8 encoding said TCR construct; or
b) a protein of claim 9 comprising said TCR construct; or
c) a host cell of claim 10 expressing said TCR construct.

12. The pharmaceutical composition of claim 11 for use in the treatment of a patient expressing HLA-A1 and having a MAGEA4-positive cancer selected from the group comprising melanoma, gastric cancer, head and neck cancer, lung cancer, breast cancer, ovarian cancer, bladder cancer, and esophageal cancer.

13. The pharmaceutical composition of claim 11 or the pharmaceutical composition for use of claim 12, wherein the treatment is an immunotherapy selected from the group comprising adoptive T cell therapy and TCR gene therapy.

14. A pharmaceutical composition comprising a peptide comprising SEQ ID NO: 1, SEQ ID NO: 18 or SEQ ID NO: 19, wherein the peptide is capable of being presented on HLA-A*01-01, and wherein the peptide comprises at most 11, preferably, at most 10 consecutive amino acids identical to the sequence of amino acids ocurring in MAGEA4 of SEQ ID NO: 14;
or comprising a nucleic acid encoding said peptide.

15. The pharmaceutical composition of claim 14 for use in vaccination against a MAGEA4-positive cancer selected from the group comprising melanoma, gastric cancer, head and neck cancer, lung cancer, breast cancer, ovarian cancer, bladder cancer, and esophageal cancer.
